# EUROPÄISCHE PATENTSCHRIFT

(11) **EP 3 402 775 B1**
(45) Veröffentlichungstag und Bekanntmachung des Hinweises auf die Patenterteilung: **09.06.2021**
(21) Anmeldenummer: 17700087.4
(22) Anmeldetag: 09.01.2017
(51) Int. Cl.: C07C 29/36, C07C 33/20, C07C 33/46, C07C 43/23

(54) **VERFAHREN ZUR HERSTELLUNG VON 2-(4-CHLOR-2,6-DIMETHYLPHENYL)ETHANOL**
METHOD FOR THE PREPARATION OF 2-(4-CHLORO-2,6-DIMETHYLPHENYL)ETHANOL
PROCÉDÉ DE FABRICATION DE 2-(4-CHLORO-2,6-DIMÉTHYLPHÉNYL)ÉTHANOL

(30) Priorität: 15.01.2016 EP 16151430
(43) Veröffentlichungstag der Anmeldung: 21.11.2018
(73) Patentinhaber: Bayer CropScience Aktiengesellschaft, 40789 Monheim am Rhein (DE)
(72) Erfinder: HIMMLER, Thomas, 51519 Odenthal (DE); BRÜCHNER, Peter, 47809 Krefeld (DE)
(74) Vertreter: BIP Patents
(86) Internationale Anmeldenummer: PCT/EP2017/050325
(87) Internationale Veröffentlichungsnummer: WO 2017/121699

(56) Entgegenhaltungen:
- WO-A1-2013/080896
- HUYNH, C. ET AL.: "Copper-catalysed reactions of grignard reagents with epoxides and oxetane.", TETRAHEDRON LETTERS, Bd. 20, Nr. 17, 1979, Seiten 1503-1506, XP055287523, ISSN: 0040-4039, DOI: 10.1016/S0040-4039(01)86190-6 in der Anmeldung erwähnt
- SCHÜPBACH, B. ET AL.: "A divergent synthesis of oligoarylalkanethiols with Lewis-basic N-donor termini", ORGANIC & BIOMOLECULAR CHEMISTRY, Bd. 8, Nr. 15, 2010, Seiten 3552-3562, XP055287525, ISSN: 1477-0520, DOI: 10.1039/c003795h in der Anmeldung erwähnt
- BOUDJOUK, P. ET AL.: "THE SYNTHESIS OF 1-SILAPHENALANES FROM 1,8-DIFUNCTIONAL NAPHTHALENES. CONFIRMATION OF THE STRUCTURES OF THE PYROLYSIS PRODUCTS OF (1-NAPHTHYL)VINYLDICHLOROSILANE", JOURNAL OF ORGANOMETALLIC CHEMISTRY, Bd. 221, Nr. 1, 1981, Seiten 33-45, XP055287726, DOI: doi:10.1016/S0022-328X(00)81026-8
- MA, W. ET AL.: "Synthesis of New Four-Atom-Linked Capped Porphyrins", THE JOURNAL OF ORGANIC CHEMISTRY, Bd. 60, Nr. 24, 1995, Seiten 8081-8083, XP055287724, ISSN: 0022-3263, DOI: 10.1021/jo00129a058
- BOST, J.J. ET AL.: "Effect of Structural Changes on Adsorption of Certain Alcohol 3,5-dinitrobenzoates on Silicic Acid", THE JOURNAL OF ORGANIC CHEMISTRY, Bd. 22, Nr. 1, 1957, Seiten 51-55, XP055287887, ISSN: 0022-3263, DOI: 10.1021/jo01352a013

## Beschreibung

Die vorliegende Erfindung betrifft ein Verfahren zur Herstellung von substituierten 2-Aryl-ethanolen, sowie neue substituierte 2-Aryl-ethanole.

Substituierte 2-Aryl-ethanole sind wichtige Intermediate für die Herstellung bioaktiver Verbindungen, die speziell zur Kontrolle von Schädlingen im Pflanzenschutz eingesetzt werden können. Insbesondere dienen sie zur Herstellung insektizider, akarizider oder herbizider cyclischer Ketoenole (beispielsweise EP-A-456 063*,* EP-A-521 334*,* EP-A-596 298*,* EP-A-613 884*,* EP-A-613 885, WO 95/01 971, WO 95/26 954, WO 95/20 572, EP-A-0 668 267, WO 96/25 395, WO 96/35 664, WO 97/01 535, WO 97/02 243, WO 97/36 868, WO 97/43275, WO 98/05638, WO 98/06721, WO 98/25928, WO 99/24437, WO 99/43649, WO 99/48869, WO 99/55673, WO 01/17972, WO 01/23354, WO 01/74770, WO 03/013249, WO 03/062244, WO 04/007448, WO 04/024 688, WO 04/065366, WO 04/080962, WO 04/111042, WO 05/044791, WO 05/044796, WO 05/048710, WO 05/049569, WO 05/066125, WO 05/092897, WO 06/000355, WO 06/029799, WO 06/056281, WO 06/056282, WO 06/089633, WO 07/048545, WO 07/073856, WO 07/096058, WO 07/121868, WO 07/140881, WO 08/067873*,* WO 08/067910, WO 08/067911, WO 08/138551, WO 09/015801, WO 09/039975, WO 09/049851, WO 09/115262, WO 10/052161, WO 10/102758, WO 10/063378, WO 10/063670, WO 10/102758, WO 11/098443, WO 11/098440, WO 11/067135, WO 11/067240, WO 12/110519), wobei aus den substituierten 2-Aryl-ethanolen die benötigten substituierten Phenylessigsäuren hergestellt werden können (WO 2013/080896).

Es ist bereits eine Vielzahl von Methoden zur Herstellung von substituierten 2-Aryl-ethanolen bekannt geworden. Eine in der Literatur häufig zu findende Methode besteht darin, substituierte Phenylessigsäureester mit einem komplexen Hydrid wie beispielsweise Lithiumaluminiumhydrid zu reduzieren (siehe beispielsweise WO 2011/123937). Man setzt hier also gerade die Verbindungen als Ausgangsmaterialien ein, die häufig nur nach aufwendigen Methoden zu erhalten sind und die gerade aus den substituierten 2-Aryl-ethanolen leichter erhältlich sein sollten.

Des Weiteren ist bekannt, einen Halogenaromaten mit Acetylen oder einem mono-geschützten Acetylen wie beispielsweise Trimethylsilyl-acetylen oder 2-Methylbut-3-in-2-ol in einer Sonogashira-Reaktion unter Palladiumkatalyse zum entsprechenden geschützten Aryl-acetylen umzusetzen, die Schutzgruppe zu entfernen und anschließend die Alkinfunktion einer Anti-Markovnikov-Addition von Wasser zu unterziehen, beispielsweise katalysiert durch Rutheniumkomplexe (J. Amer. Chem. Soc. 136 (2014) 7058-67). Diese Methode hat den Nachteil, zwei übergangsmetallkatalysierte Schritte zu enthalten, einer davon mit dem sehr teuren Metall Palladium.

Eine weitere bekannte Methode besteht darin, einen Halogenaromaten mit Butyllithium zu metallieren und anschließend mit Ethylenoxid umzusetzen (siehe beispielsweise Eur. J. Med. Chem. 25 (1990) 603-8). Dieses Verfahren hat den Nachteil, dass die Metallierung mit Butyllithium in der Regel bei sehr tiefen Temperaturen, beispielsweise -78°C, durchgeführt werden muß, was technisch nur aufwendig und teuer zu realisieren ist und somit den Prozess unökonomisch macht.

Eine weitere bekannte Methode besteht darin, einen Halogenaromaten mit Magnesium zu der entsprechenden Grignard-Verbindung umzusetzen und diese Grignard-Verbindung dann mit Ethylenoxid zu dem substituierten 2-Aryl-ethanol reagieren zu lassen. Diese Methode hat den Nachteil, dass unter Umständen ein Gemisch aus dem gewünschten substituierten 2-Aryl-ethanol und unerwünschtem substituierten 1-Aryl-ethanol erhalten wird (siehe Vergleichsversuche), wodurch aufwendige Reinigungsoperationen notwendig werden und die Ausbeute unbefriedigend wird.

Es ist zwar bereits beschrieben worden, dass die Reaktion von Grignard-Verbindungen mit Oxiranen, darunter auch Ethylenoxid, durch Zusatz von Kupferverbindungen wie Kupferiodid beschleunigt werden kann (siehe beispielsweise Tetrahedron Letters 1978, 4069-72*;* Tetrahedron Letters 1979, 1503-6*;* Org. Biomol. Chem. 2010, 3552-62). Es ist allerdings bislang kein Hinweis bekanntgeworden, dass auch die Selektivität im gewünschten Sinne verbessert werden kann.

Ebenso ist bekannt, dass man 2-Arylethanole dadurch erhält, dass eine Aryl-Grignard-Verbindung mit 2-Bromethanol umgesetzt wird, ebenfalls in Gegenwart einer katalytischen Menge von Kupfer(I)bromid *(*Tetrahedron Letters 1977, 3263-66). Sehr nachteilig ist hierbei der Umstand, dass die Grignard-Verbindung in einem sehr hohen Überschuß von 3 Moläquivalenten eingesetzt wird, wodurch dieses Verfahren unwirtschaftlich wird.

Es besteht dementsprechend weiterhin ein hoher Bedarf an einem verbesserten Verfahren zur Herstellung von substituierten 2-Aryl-ethanolen.

Es wurde nun gefunden, dass bei der Reaktion von Grignard-Verbindungen mit Ethylenoxid die Bildung von unerwünschten 1-Aryl-ethanolen überraschenderweise durch Zusatz katalytischer Mengen von Kupfersalzen unterdrückt werden kann.

Die vorliegende Erfindung beinhaltet daher ein neues Verfahren zur Herstellung von substituierten 2-Aryl-ethanolen der Formel (I) 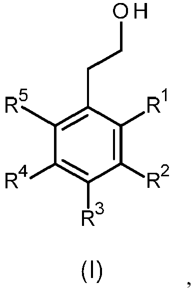 in welcher
R¹ für Methyl steht,
R² für Wasserstoff steht,
R³ für Chlor steht,
R⁴ für Wasserstoff steht,
R⁵ für Methyl steht, dadurch gekennzeichnet, dass eine Grignard-Verbindung der Formel (II) 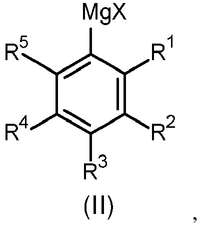 in der die Reste R¹ bis R⁵ die oben angegebenen Bedeutungen haben und
X für Chlor, Brom oder Iod (bevorzugt für Brom oder Iod, besonders bevorzugt für Brom) steht, in Gegenwart einer Kupferverbindung mit Ethylenoxid umgesetzt wird, wobei als Kupferverbindung Kupfer(I)-iodid, Kupfer(I)-bromid, Kupfer(II)-bromid und Kupfer(I)-chlorid eingesetzt wird.

Mit der Verbindung der Formel (II) sollen auch die dem Fachmann bekannten anderen Formen des Schlenk-Gleichgewichtes, mit und ohne Komplexierung von Lösungsmittelmolekülen, mit umfasst sein. Hervorgehoben ist die Herstellung von 2-(4-Chlor-2,6-dimethylphenyl)ethanol.

Die Herstellung der Grignard-Verbindungen der Formel (II) erfolgt nach allgemein bekannten Methoden der organischen Chemie aus dem entsprechenden substituierten Arylhalogenid und Magnesium. Als Arylhalogenide können dabei die Chlor-, Brom- oder Iodaromaten eingesetzt werden. Bevorzugt verwendet man die Brom- und Iodaromaten, besonders bevorzugt die Bromaromaten.

Als Lösungsmittel bei der Herstellung der Grignard-Verbindungen der Formel (II) kommen beispielsweise offenkettige und cyclische Ether in Frage wie beispielsweise Diethylether, Methyl-tertiärbutylether, Tertiäramyl-methyl-ether, Tetrahydrofuran, 2-Methyl-tetrahydrofuran, 2,5-Dimethyl-tetrahydrofuran, Methyl-cyclopentyl-ether oder 1,4-Dioxan; aromatische Kohlenwasserstoffe wie Toluol, Xylole oder Mesitylen; Gemische dieser Lösungsmittel. Bevorzugt arbeitet man in cyclischen Ethern oder in Gemischen cyclischer Ether mit aromatischen Kohlenwasserstoffen.

Die Temperatur bei der Herstellung der Grignard-Verbindungen der Formel (II) kann in weiten Grenzen variieren. Bevorzugt arbeitet man zwischen 20°C und 100°C.

Das Magnesium wird in der Regel in einem Überschuß bezogen auf den Halogenaromaten eingesetzt, üblicherweise 1,05 bis 1,2 Äquivalente.

Nach erfolgter Umsetzung des Halogenaromaten mit dem Magnesium kann das nicht abreagierte überschüssige Magnesium durch eine Filtration entfernt werden.

Im erfindungsgemäßen Schritt des Verfahrens wird die wie oben beschrieben hergestellte Grignard-Verbindung der Formel (II) in Gegenwart einer Kupferverbindung mit Ethylenoxid umgesetzt.

Als Lösungsmittel für den erfindungsgemäße Schritt des Verfahrens kommen die Lösungsmittel in Frage, die zur Herstellung der Grignard-Verbindung der Formel (II) verwendet werden: Offenkettige und cyclische Ether wie Diethylether, Methyl-tertiärbutylether, Tertiäramyl-methyl-ether, Tetrahydrofuran, 2-Methyl-tetrahydrofuran, 2,5-Dimethyl-tetrahydrofuran, Methyl-cyclopentyl-ether oder 1,4-Dioxan; aromatische Kohlenwasserstoffe wie Toluol, Xylole oder Mesitylen; Gemische dieser Lösungsmittel. Bevorzugt arbeitet man in cyclischen Ethern oder in Gemischen cyclischer Ether mit aromatischen Kohlenwasserstoffen. Besonders bevorzugt sind Tetrahydrofuran, 2-Methyl-tetrahydrofuran, Methyl-cyclopentyl-ether, Gemische dieser Ether und Gemische dieser Ether mit Toluol.

Als Kupferverbindungen werden im erfindungsgemäßen Schritt des Verfahrens Kupfer(I)- oder Kupfer(II)-Verbindungen eingesetzt. Beispielhaft seien genannt Kupfer(I)-iodid, Kupfer(I)-bromid, Kupfer(I)-chlorid, Kupfer(I)-oxid, Kupfer(II)-bromid, Kupfer(II)-chlorid, Kupfer(II)-oxid, Kupfer(II)-sulfat, Kupfer(II)-nitrat, Kupfer(II)-acetat. Bevorzugt verwendet man Kupfer(I)-iodid, Kupfer(I)-bromid, Kupfer(II)-bromid und Kupfer(I)-chlorid, besonders bevorzugt Kupfer(I)-iodid Kupfer(I)-bromid und Kupfer(II)-bromid.

Die Menge an Kupferverbindung im erfindungsgemäßen Schritt des Verfahrens kann in weiten Grenzen variiert werden. Bevorzugt verwendet man die geringste notwendige Menge an Kupfer-Verbindung, mit der der gewünschte Effekt bewirkt werden kann. Bevorzugt setzt man 0,1 bis 50 Molprozent, bezogen auf die Grignard-Verbindung der Formel (II) ein; besonders bevorzugt 0,5 bis 15 Molprozent.

Die Menge an Ethylenoxid im erfindungsgemäßen Schritt des Verfahrens kann ebenfalls in weiten Grenzen variiert werden. Bevorzugt verwendet man zwischen 0,9 und 3 Moläquivalente Ethylenoxid, bezogen auf die Grignard-Verbindung der Formel (II). Besonders bevorzugt verwendet man zwischen 1 und 2 Moläquivalente Ethylenoxid.

Das Ethylenoxid kann entweder als Gas in die Lösung der Grignard-Verbindung der Formel (II) eingeleitet werden, oder man dosiert das Ethylenoxid als Lösung zu. Als Lösungsmittel kommen dabei bevorzugt diejenigen Lösungsmittel in Frage, die bei der Herstellung der Grignard-Verbindung der Formel (II) verwendet wurden.

Die Reaktionstemperatur im erfindungsgemäßen Schritt des Verfahrens liegt zwischen -30 und + 100°C. Bevorzugt liegt sie zwischen 0 und 80°C, besonders bevorzugt zwischen +10 und +50°C.

Die Reaktion im erfindungsgemäßen Schritt des Verfahrens kann prinzipiell auch unter vermindertem oder erhöhtem Druck durchgeführt werden. Bevorzugt arbeitet man bei Normaldruck.

Die Aufarbeitung der Reaktionsgemische erfolgt nach üblichen und bekannten Methoden der organischen Chemie.

Folgende substituierte 2-Aryl-ethanole der Formel (I) sind genannt 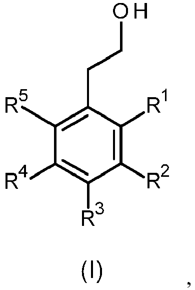 in der die Reste R¹, R², R³, R⁴, R⁵ die in der Tabelle 1 angegebenen Bedeutungen haben.

**Tabelle 1**

| **Verbindung** | **R¹** | **R²** | **R³** | **R⁴** | **R⁵** |
|---|---|---|---|---|---|
| **I-1** | **Me** | **H** | **Cl** | **H** | **Me** |
| **I-2** | **Me** | **H** | **Cl** | **H** | **Et** |
| **I-3** | **Et** | **H** | **Cl** | **H** | **Et** |
| **I-4** | **Me** | **H** | **Cl** | **H** | ***n*-Pr** |
| **I-5** | **Et** | **H** | **Cl** | **H** | ***n*-Pr** |
| **I-6** | ***n*-Pr** | **H** | **Cl** | **H** | ***n*-Pr** |
| **I-7** | **Me** | **H** | **Cl** | **H** | ***iso*-Pr** |
| **I-8** | **Et** | **H** | **Cl** | **H** | ***iso*-Pr** |
| **I-9** | ***iso-Pr*** | **H** | **Cl** | **H** | ***iso*-Pr** |
| **I-10** | ***iso-Pr*** | **H** | **Cl** | **H** | ***n*-Pr** |
| **I-11** | **Me** | **H** | **F** | **H** | **Me** |
| **I-12** | **Me** | **H** | **F** | **H** | **Et** |
| **I-13** | **Et** | **H** | **F** | **H** | **Et** |
| **I-14** | **Me** | **H** | **CF₃** | **H** | **Me** |
| **I-15** | **Me** | **H** | **CF₃** | **H** | **Et** |
| **I-16** | **Et** | **H** | **CF₃** | **H** | **Et** |
| **I-17** | **Me** | **H** | **OCF₃** | **H** | **Me** |
| **I-18** | **Me** | **H** | **Me** | **H** | **Et** |
| **I-19** | **Me** | **H** | **Et** | **H** | **Et** |
| **I-20** | **Me** | **H** | **Et** | **H** | **Me** |
| **I-21** | **Me** | **Cl** | **H** | **H** | **Me** |
| **I-22** | **Et** | **Cl** | **H** | **H** | **Me** |
| **I-23** | **Et** | **H** | **H** | **Cl** | **Me** |

| | | | | | |
|---|---|---|---|---|---|
| Me = Methyl, Et = Ethyl, *n*-Pr = *n*-Propyl, *iso*-Pr = *iso*-Propyl | | | | | |

Besonders bevorzugt sind substituierte 2-Aryl-ethanole der Formel (I), in der die Reste R¹, R², R³, R⁴, R⁵ die in der **Tabelle 2** angegebenen Bedeutungen haben.

**Tabelle 2**

| **Verbindung** | **R¹** | **R²** | **R³** | **R⁴** | **R⁵** |
|---|---|---|---|---|---|
| **I-1** | **Me** | **H** | **Cl** | **H** | **Me** |
| **I-2** | **Me** | **H** | **Cl** | **H** | **Et** |
| **I-3** | **Et** | **H** | **Cl** | **H** | **Et** |
| **I-4** | **Me** | **H** | **Cl** | **H** | ***n*-Pr** |
| **I-5** | **Et** | **H** | **Cl** | **H** | ***n*-Pr** |
| **I-6** | ***n*-Pr** | **H** | **Cl** | **H** | ***n*-Pr** |
| **I-11** | **Me** | **H** | **F** | **H** | **Me** |
| **I-12** | **Me** | **H** | **F** | **H** | **Et** |
| **I-13** | **Et** | **H** | **F** | **H** | **Et** |
| **I-18** | **Me** | **H** | **Me** | **H** | **Et** |
| **I-19** | **Me** | **H** | **Et** | **H** | **Et** |
| **I-20** | **Me** | **H** | **Et** | **H** | **Me** |
| **I-21** | **Me** | **Cl** | **H** | **H** | **Me** |
| **I-22** | **Et** | **Cl** | **H** | **H** | **Me** |
| **I-23** | **Et** | **H** | **H** | **Cl** | **Me** |

Ganz besonders bevorzugt sind substituierte 2-Aryl-ethanole der Formel (I), in der die Reste R¹, R², R³, R⁴, R⁵ die in der **Tabelle 3** angegebenen Bedeutungen haben.

**Tabelle 3**

| **Verbindung** | **R¹** | **R²** | **R³** | **R⁴** | **R⁵** |
|---|---|---|---|---|---|
| **I-1** | **Me** | **H** | **Cl** | **H** | **Me** |
| **I-2** | **Me** | **H** | **Cl** | **H** | **Et** |
| **I-3** | **Et** | **H** | **Cl** | **H** | **Et** |
| **I-11** | **Me** | **H** | **F** | **H** | **Me** |
| **I-12** | **Me** | **H** | **F** | **H** | **Et** |
| **I-13** | **Et** | **H** | **F** | **H** | **Et** |
| **I-18** | **Me** | **H** | **Me** | **H** | **Et** |
| **I-19** | **Me** | **H** | **Et** | **H** | **Et** |
| **I-20** | **Me** | **H** | **Et** | **H** | **Me** |
| **I-21** | **Me** | **Cl** | **H** | **H** | **Me** |

Darüber hinaus gehend bevorzugt ist folgende Verbindung:
2-(4-Chlor-2,6-dimethylphenyl)ethanol (Verbindung I-1).

Die Oxidation der substituierten 2-Aryl-ethanole der Formel (I) zu den als Bausteine beispielsweise für insektizide oder herbizide cyclische Ketoenole benötigten substituierten Phenylessigsäuren kann nach grundsätzlich bekannten Methoden der organischen Chemie erfolgen. Beispielhaft genannt seien die Oxidation mit Kaliumpermanganat oder die Zhao-Anelli-Oxidation mit 2,2,6,6-Tetramethylpiperidinyloxyl, Natriumhypochlorit und Natriumchlorit (Organic Syntheses, 81, 195-203; 2005).

Die vorliegende Erfindung soll durch folgende Beispiele näher erläutert werden.

### Beispiele

### Beispiel 1: 2-(4-Chlor-2,6-dimethylphenyl)ethanol (Verbindung I-1)

Zu einer Lösung von Brom(4-chlor-2,6-dimethylphenyl)magnesium, hergestellt bei 30 - 35°C aus 50 mmol 4-Chlor-2,6-dimethyl-brombenzol, 1 mmol Brom(4-chlor-2,6-dimethylphenyl)magnesium (zum Starten der Grignard-Synthese) und 55,5 mmol Magnesium in 50 ml Tetrahydrofuran, wurden 5 mmol Kupfer(I)iodid gegeben. Anschließend wurden 48 ml einer 2,5 - 3,3 molaren Lösung von Ethylenoxid in Tetrahydrofuran (120 mmol, berechnet für eine Konzentration von 2,5 M) bei 20°C innerhalb von 30 Minuten zudosiert. Nach 16 Stunden bei 20°C wurde das Reaktionsgemisch auf 100 g Eis gegeben und mit Schwefelsäure auf pH 1 gestellt. Nach dreimaliger Extraktion mit je 50 ml Methylenchlorid wurden die vereinigten organischen Phasen einmal mit 30 ml Wasser ausgeschüttelt, über Magnesiumsulfat getrocknet und einrotiert. Es verblieb ein Öl, in dem nach GC/MS-Analyse das Verhältnis von 2-(4-Chlor-2,6-dimethylphenyl)ethanol zu 1-(4-Chlor-2,6-dimethylphenyl)ethanol >99 : 1 war.
GC/MS: m/e = 184 (M⁺ (³⁵Cl), 25%), 153 (³⁵Cl, 100%).
¹H-NMR (600 MHz, d-DSMO): δ = 2,28 (s, 6H), 2,75 (m, 2H), 3,45 (m, 2H), 4,74 (m, 1H), 7,0 (s, 2H)ppm.

### Beispiel 2: 2-(4-Chlor-2,6-dimethylphenyl)ethanol (Verbindung I-1)

Zu einer Lösung von Brom(4-chlor-2,6-dimethylphenyl)magnesium, hergestellt bei 30 - 35°C aus 10 mmol 4-Chlor-2,6-dimethyl-brombenzol, 1 mmol Brom(4-chlor-2,6-dimethylphenyl)magnesium (zum Starten der Grignard-Synthese) und 11,1 mmol Magnesium in 10 ml Tetrahydrofuran, wurden 0,1 mmol Kupfer(I)iodid gegeben. Anschließend wurden 9,6 ml einer 2,5 - 3,3 molaren Lösung von Ethylenoxid in Tetrahydrofuran (24 mmol, berechnet für eine Konzentration von 2,5 M) bei 20°C innerhalb von 30 Minuten zudosiert. Nach 16 Stunden bei 20°C wurde das Reaktionsgemisch auf 100 g Eis gegeben und mit Schwefelsäure auf pH 1 gestellt. Nach dreimaliger Extraktion mit je 50 ml Methylenchlorid wurden die vereinigten organischen Phasen einmal mit 30 ml Wasser ausgeschüttelt, über Magnesiumsulfat getrocknet und einrotiert. Es verblieb ein Öl, in dem nach GC/MS-Analyse das Verhältnis von 2-(4-Chlor-2,6-dimethylphenyl)ethanol zu 1-(4-Chlor-2,6-dimethylphenyl)ethanol >99 : 1 war.

### Beispiel 3: 2-(4-Chlor-2,6-dimethylphenyl)ethanol (Verbindung I-1)

Zu einer Lösung von Brom(4-chlor-2,6-dimethylphenyl)magnesium, hergestellt bei 30 - 35°C aus 10 mmol 4-Chlor-2,6-dimethyl-brombenzol, 1 mmol Brom(4-chlor-2,6-dimethylphenyl)magnesium (zum Starten der Grignard-Synthese) und 11,1 mmol Magnesium in 10 ml Tetrahydrofuran, wurden 1 mmol Kupfer(I)iodid gegeben. Anschließend wurden 9,6 ml einer 2,5 - 3,3 molaren Lösung von Ethylenoxid in Tetrahydrofuran (24 mmol, berechnet für eine Konzentration von 2,5 M) bei 50°C innerhalb von 30 Minuten zudosiert. Nach 16 Stunden bei 50°C wurde das Reaktionsgemisch auf 100 g Eis gegeben und mit Schwefelsäure auf pH 1 gestellt. Nach dreimaliger Extraktion mit je 50 ml Methylenchlorid wurden die vereinigten organischen Phasen einmal mit 30 ml Wasser ausgeschüttelt, über Magnesiumsulfat getrocknet und einrotiert. Es verblieb ein Öl, in dem nach GC/MS-Analyse das Verhältnis von 2-(4-Chlor-2,6-dimethylphenyl)ethanol zu 1-(4-Chlor-2,6-dimethylphenyl)ethanol >99 : 1 war.

### Vergleichsbeispiel 1: 2-(4-Chlor-2,6-dimethylphenyl)ethanol

Zu einer Lösung von Brom(4-chlor-2,6-dimethylphenyl)magnesium, hergestellt bei 30 - 50°C aus 10 mmol 4-Chlor-2,6-dimethyl-brombenzol und 11,1 mmol Magnesium in 10 ml Tetrahydrofuran, wurden 8,8 ml einer 2,5 - 3,3 molaren Lösung von Ethylenoxid in Tetrahydrofuran (22 mmol, berechnet für eine Konzentration von 2,5 M) bei 50°C innerhalb von 30 Minuten zudosiert. Nach 3 Stunden bei 50°C wurde das Reaktionsgemisch auf 100 g Eis gegeben und mit Schwefelsäure auf pH 1 gestellt. Nach dreimaliger Extraktion mit je 50 ml Methylenchlorid wurden die vereinigten organischen Phasen einmal mit 30 ml Wasser ausgeschüttelt, über Magnesiumsulfat getrocknet und einrotiert. Es verblieb ein Öl, in dem nach GC/MS-Analyse das Verhältnis von 2-(4-Chlor-2,6-dimethylphenyl)ethanol zu 1-(4-Chlor-2,6-dimethylphenyl)ethanol 87 : 13 betrug.

### Vergleichsbeispiel 2 : 2-(4-Chlor-2,6-dimethylphenyl)ethanol

Zu einer Lösung von Brom(4-chlor-2,6-dimethylphenyl)magnesium, hergestellt bei 30 - 35°C aus 10 mmol 4-Chlor-2,6-dimethyl-brombenzol, 1 mmol Brom(4-chlor-2,6-dimethylphenyl)magnesium (zum Starten der Grignard-Synthese) und 11,1 mmol Magnesium in 10 ml Tetrahydrofuran, wurden 9,6 ml einer 2,5 - 3,3 molaren Lösung von Ethylenoxid in Tetrahydrofuran (24 mmol, berechnet für eine Konzentration von 2,5 M) bei 50°C innerhalb von 30 Minuten zudosiert. Nach 16 Stunden bei 50°C wurde das Reaktionsgemisch auf 100 g Eis gegeben und mit Schwefelsäure auf pH 1 gestellt. Nach dreimaliger Extraktion mit je 50 ml Methylenchlorid wurden die vereinigten organischen Phasen einmal mit 30 ml Wasser ausgeschüttelt, über Magnesiumsulfat getrocknet und einrotiert. Es verblieb ein Öl, in dem nach GC/MS-Analyse das Verhältnis von 2-(4-Chlor-2,6-dimethylphenyl)ethanol zu 1-(4-Chlor-2,6-dimethylphenyl)ethanol 78 : 22 betrug.

### Beispiel 4: 2-(4-Chlor-2,6-dimethylphenyl)ethanol (Verbindung I-1)

Zu einer Lösung von 20 mmol Brom(4-chlor-2,6-dimethylphenyl)magnesium in 20 ml Tetrahydrofuran, wurden 2 mmol Kupfer(I)bromid gegeben. Anschließend wurden 16 ml einer 2,5 - 3,3 molaren Lösung von Ethylenoxid in Tetrahydrofuran (40 mmol, berechnet für eine Konzentration von 2,5 M) bei 20°C innerhalb von 30 Minuten zudosiert. Nach 16 Stunden bei 20°C wurde das Reaktionsgemisch auf 100 g Eis gegeben und mit Schwefelsäure auf pH 1 gestellt. Nach dreimaliger Extraktion mit je 50 ml Methylenchlorid wurden die vereinigten organischen Phasen einmal mit 30 ml Wasser ausgeschüttelt, über Magnesiumsulfat getrocknet und einrotiert. Es verblieb ein Öl, in dem nach GC/MS-Analyse das Verhältnis von 2-(4-Chlor-2,6-dimethylphenyl)ethanol zu 1-(4-Chlor-2,6-dimethylphenyl)ethanol >99 : 1 betrug.

### Beispiel 5: 2-(4-Chlor-2,6-dimethylphenyl)ethanol (Verbindung I-1)

Man verfuhr wie in Beispiel 4, setzte jedoch anstelle von Cu(I)Br nun 1 mmol Kupfer(II)bromid ein. Das Verhältnis von 2-(4-Chlor-2,6-dimethylphenyl)ethanol zu 1-(4-Chlor-2,6-dimethylphenyl)ethanol war >99 : 1.

### Beispiel 6: 2-(4-Chlor-2,6-dimethylphenyl)ethanol (Verbindung I-1)

Man verfuhr wie in Beispiel 4, setzte jedoch anstelle von Cu(I)Br nun 1 mmol Kupfer(I)chlorid ein. Das Verhältnis von 2-(4-Chlor-2,6-dimethylphenyl)ethanol zu 1-(4-Chlor-2,6-dimethylphenyl)ethanol war >99 : 1.

### Beispiel 7: 2-(2,6-Dimethylphenyl)ethanol nicht erfindungsgemäß

Zu einer Lösung von 2,6-Dimethylphenylmagnesium, hergestellt bei 30 - 35°C aus 20 mmol 2,6-Dimethyl-brombenzol und 22,2 mmol Magnesium in 10 ml Tetrahydrofuran, wurden 0,2 mmol Kupfer(I)iodid gegeben. Anschließend wurden 8,8 ml einer 2,5 - 3,3 molaren Lösung von Ethylenoxid in Tetrahydrofuran (22 mmol, berechnet für eine Konzentration von 2,5 M) bei 20°C innerhalb von 30 Minuten zudosiert. Nach 16 Stunden bei 20°C wurde das Reaktionsgemisch auf 100 g Eis gegeben und mit Schwefelsäure auf pH 1 gestellt. Nach dreimaliger Extraktion mit je 50 ml Methylenchlorid wurden die vereinigten organischen Phasen einmal mit 30 ml Wasser ausgeschüttelt, über Magnesiumsulfat getrocknet und einrotiert. Es verblieb ein Öl, in dem nach GC/MS-Analyse das Verhältnis von 2-(2,6-dimethylphenyl)ethanol zu 1-(2,6-Dimethylphenyl)ethanol 97,5 : 2,5 betrug.

### Vergleichsbeispiel 3: 2-(2,6-Dimethylphenyl)ethanol

In eine Lösung von 2,6-Dimethylphenylmagnesium, hergestellt bei 40 - 55°C, gegen Ende für wenige Minuten bei 65°C, aus 200 mmol 2,6-Dimethyl-brombenzol und 222 mmol Magnesium in 100 ml Tetrahydrofuran, wurden bei 30 - 35°C innerhalb von etwa 2 Stunden 215 mmol Ethylenoxid geleitet. Nach 3 Stunden bei 60°C wurde das Reaktionsgemisch auf 200 g Eis gegeben und mit Schwefelsäure auf pH 1 gestellt. Nach dreimaliger Extraktion mit je 50 ml Methylenchlorid wurden die vereinigten organischen Phasen einmal mit 30 ml Wasser ausgeschüttelt, über Magnesiumsulfat getrocknet und einrotiert. Es verblieb ein Öl, in dem nach GC/MS-Analyse das Verhältnis von 2-(2,6-dimethylphenyl)ethanol zu 1-(2,6-Dimethylphenyl)ethanol 81 : 19 betrug.

### Verwendungsbeispiel 1: 4-Chlor-2,6-dimethylphenylessigsäure

Zu einer Lösung von 5g 2-(4-Chlor-2,6-dimethylphenyl)ethanol (24mmol, Reinheit 90%) in 20g Acetonitril wurden 38mg 2,2,6,6-Tetramethylpiperidinyloxyl (0,24mmol) bei Raumtemperatur gegeben. Zu dieser Lösung gab man bei 45°C 0,8ml 11,05%ige Natriumhypochloritlösung und anschließend wurden 4,3g Natriumchlorit (36mmol), gelöst in 12,5g eines Phosphatpuffers (10,65g Na₂HPO₄ und 10,21g KH₂PO₄ auf 1000ml Wasser) innerhalb einer Stunde mittels Dosierpumpe zugetropft. Nach beendeter Zugabe wurde 30min nachgerührt, auf 5-10°C abgekühlt und 3g Natriumsulfit portionsweise addiert. Die Reaktionsmischung wurde dann für eine Stunde nachgerührt, mit 45%iger Natronlauge auf pH 13,5 gestellt und die resultierende Suspension zweimal mit je 25ml MTBE extrahiert. Die wässrige Phase wurde mit 10%iger Salzsäure auf pH 3,38 gestellt und dreimal mit je 30ml MTBE extrahiert. Die vereinigten organischen Phasen der Säureextraktion wurden getrocknet und eingeengt. Man erhielt 4,3g Produkt (87% der Theorie; Reinheit 98% nach HPLC und quant. NMR).

## Patentansprüche

1. Verfahren zur Herstellung von Verbindungen der Formel (I) 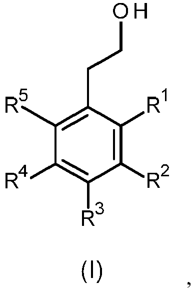 in welcher
R¹ für Methyl steht,
R² für Wasserstoff steht,
R³ für Chlor steht,
R⁴ für Wasserstoff steht,
R⁵ für Methyl steht,
**dadurch gekennzeichnet, dass** eine Verbindung der Formel (II) 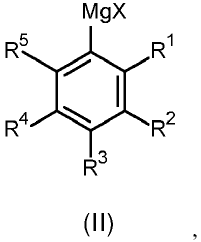 in der die Reste R¹ bis R⁵ die oben angegebenen Bedeutungen haben und
X für Chlor, Brom oder Iod steht,
in Gegenwart einer Kupferverbindung mit Ethylenoxid umgesetzt wird, wobei als Kupferverbindung Kupfer(I)-iodid, Kupfer(I)-bromid, Kupfer(II)-bromid oder Kupfer(I)-chlorid eingesetzt wird .

2. Verfahren zur Herstellung von Verbindungen der Formel (I) gemäß Anspruch 1, **dadurch gekennzeichnet, dass** die Kupferverbindung in Mengen von 0,1 bis 50 Molprozent bezogen auf die Verbindung der Formel (II) eingesetzt wird.

3. Verfahren zur Herstellung von Verbindungen der Formel (I) gemäß Anspruch 1, **dadurch gekennzeichnet, dass** die Kupferverbindung in Mengen vom 0,5 bis 15 Molprozent bezogen auf die Verbindung der Formel (II) eingesetzt wird.

4. Verfahren zur Herstellung von Verbindungen der Formel (I) gemäß Anspruch 1, **dadurch gekennzeichnet, dass** Ethylenoxid in Mengen zwischen 0,9 und 3 Moläquivalente bezogen auf die Verbindung der Formel (II) eingesetzt wird.

5. Verfahren zur Herstellung von Verbindungen der Formel (I) gemäß Anspruch 1, **dadurch gekennzeichnet, dass** Ethylenoxid in Mengen zwischen 1 und 2 Moläquivalente bezogen auf die Verbindung der Formel (II) eingesetzt wird.

## Claims

1. Method for preparing compounds of the formula (I) 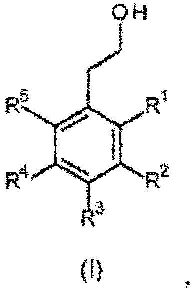 in which
R¹ is methyl,
R² is hydrogen,
R³ is chlorine,
R⁴ is hydrogen,
R⁵ is methyl,
**characterized in that** a compound of the formula (II) 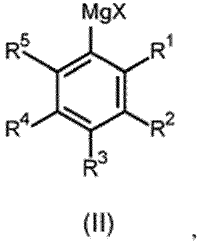 in which the radicals R¹ to R⁵ have the definitions specified above and
X is chlorine, bromine or iodine,
is reacted with ethylene oxide in the presence of a copper compound, wherein the copper compound used is copper(I) iodide, copper(I) bromide, copper(II) bromide or copper (I) chloride.

2. Method for preparing compounds of the formula (I) according to Claim 1, **characterized in that** the copper compound is used in amounts of 0.1 to 50 mole percent, based on the compound of the formula (II).

3. Method for preparing compounds of the formula (I) according to Claim 1, **characterized in that** the copper compound is used in amounts of 0.5 to 15 mole percent, based on the compound of the formula (II).

4. Method for preparing compounds of the formula (I) according to Claim 1, **characterized in that** ethylene oxide is used in amounts between 0.9 and 3 mole equivalents, based on the compound of the formula (II).

5. Method for preparing compounds of the formula (I) according to Claim 1, **characterized in that** ethylene oxide is used in amounts between 1 and 2 mole equivalents, based on the compound of the formula (II).

## Revendications

1. Procédé pour la préparation de composés de formule (I) 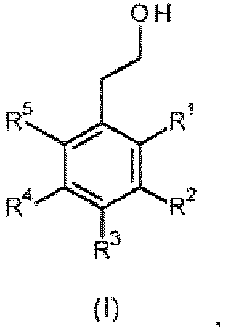 dans laquelle
R¹ représente méthyle,
R² représente hydrogène,
R³ représente chlore,
R⁴ représente hydrogène,
R⁵ représente méthyle,
**caractérisé en ce qu'**un composé de formule (II) 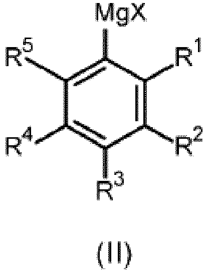 dans laquelle les radicaux R¹ à R⁵ possèdent les significations mentionnées ci-dessus et
X représente chlore, brome ou iode,
est transformé en présence d'un composé du cuivre avec de l'oxyde d'éthylène, de l'iodure de cuivre(I), du bromure de cuivre(I), du bromure de cuivre (II) ou du chlorure de cuivre(I) étant utilisé en tant que composé du cuivre.

2. Procédé pour la préparation de composés de formule (I) selon la revendication 1, **caractérisé en ce que** le composé du cuivre est utilisé en des quantités de 0,1 à 50 pour cent en moles par rapport au composé de formule (II).

3. Procédé pour la préparation de composés de formule (I) selon la revendication 1, **caractérisé en ce que** le composé du cuivre est utilisé en des quantités de 0,5 à 15 pour cent en moles par rapport au composé de formule (II).

4. Procédé pour la préparation de composés de formule (I) selon la revendication 1, **caractérisé en ce que** l'oxyde d'éthylène est utilisé en des quantités comprises entre 0,9 et 3 équivalents en moles par rapport au composé de formule (II).

5. Procédé pour la préparation de composés de formule (I) selon la revendication 1, **caractérisé en ce que** l'oxyde d'éthylène est utilisé en des quantités comprises entre 1 et 2 équivalents en moles par rapport au composé de formule (II).
